# EUROPEAN PATENT SPECIFICATION

(11) **EP 1 435 771 B1**
(45) Date of publication and mention of the grant of the patent: **01.07.2015**
(21) Application number: 02749265.1
(22) Date of filing: 16.07.2002
(51) Int. Cl.: A01N 59/00, A61L 2/16, A61L 2/20, A61L 2/23

(54) **STABLE READY-TO-USE DOSAGE FORMS CONTAINING COLORING MATTER AND ACTIVE CHLORINE, AND METHODS OF MAKING AND USING**
STABILE EINSATZBEREITE DOSIERFORMEN MIT FARBSTOFF UND AKTIVCHLOR SOWIE VERFAHREN ZU IHRER HERSTELLUNG UND VERWENDUNG
FORMULATIONS STABLES PRETES A L'EMPLOI RENFERMANT UNE SUBSTANCE COLORANTE ET DU CHLORE ACTIF, ET LEURS PROCEDES DE FABRICATION ET LEUR UTILISATION

(30) Priority: 26.07.2001 IL 14458101
(43) Date of publication of application: 14.07.2004
(73) Proprietor: Freedman Ben-Horin, Shimon, 46 325 Herzlia (IL); Lipsicas, Leon, 78 322 Ashkelon (IL)
(72) Inventor: Freedman Ben-Horin, Shimon, 46 325 Herzlia (IL); Lipsicas, Leon, 78 322 Ashkelon (IL)
(74) Representative: Dennemeyer & Associates S.A.
(86) International application number: PCT/IL2002/000574
(87) International publication number: WO 2003/009801

(56) References cited:
- WO-A-89/05093
- US-A- 4 025 628
- US-A- 4 536 367
- US-A- 4 945 110
- US-A- 5 049 385
- US-A- 5 261 353
- US-A- 5 685 262
- US-A- 5 688 435

## Description

### FIELD AND BACKGROUND OF THE INVENTION

The present invention relates to stable, ready-to-use dosage forms containing coloring matter and active chlorine. The invention also relates to methods of making such dosage forms and for using them as disinfectants.

Compounds containing (latently) active chlorine are widely used as disinfectants in various medical, veterinary, food and agricultural applications. Since compounds containing chlorine are applied to various surfaces, equipment and biological tissues as disinfectants, it would be highly desirable to color the compounds containing the active chlorine both to serve as a marker that the liquid has been applied to the respective surface as a disinfectant and also to show when the colored disinfecting compound is absent, and further, as a safety measure, to indicate that the chloroxidizer is no longer present in the vehicle. The color (e.g., a dye) is not for the purpose of indicating the level of chlorine available in the compound produced.

Solutions and compounds containing active chlorine are high oxidizing in their chemical behavior. Dyes, stains and pigments can not withstand the oxidizing effect of such chloroxidizing solutions and compounds. Dyes, stains and pigments have their chemical structures altered by oxidation and thus lose their coloring properties. This process also causes a loss in the potency of the chloroxidizer, by quantitatively reducing its oxidizing capacity.

Organic or inorganic soluble and/or insoluble salts that are colored in nature are generally maximally oxidized. Theoretically, therefore, they are stable in a solution with oxidizing properties and accordingly maintain their chemical structure and color even in the presence of such chloroxidizers. This is demonstrated, for example, by the coloration of hypochlorite solutions at a high pH with potassium permanganate (see US Patent No. 5,685,262). However, in many applications it is desirable to use a colored chloroxidizer for disinfectant purposes.

Many processes and formulations have been made for preparing colored disinfectants for use in various applications, as described for example in US Patents 5,049,385, 5,261,353, 4,025,628, 4,536,367 and 5,688,435. US US Patent 5,049,385 solid formulations comprising a particulate halogen-containing material such as calcium hypochlorite and a particulate inorganic water soluble salt such as sodium chloride, with a water dispersible colorant such as a phthalocyanine mixed into the inorganic salt, which colorant resists oxidation by the halogen-containing compound.

### OBJECTS AND BRIEF SUMMARY OF THE INVENTION

One object of the present invention is to provide a method of producing a colored disinfecting dosage form, such as a cream, ointment, paste, or gel, of an organic chloroxidizer containing latently available chlorine suitable for disinfectant purposes in which the colored appearance of the product is maintained over a period of time sufficient to complete the disinfecting or sterilizing action, without affecting the stability of the chlorine disinfectant in the product.

Another object of the invention is to provide a method of indicating by color which surface or biological tissue, such as teats of a milking animal, or skin area before a medical procedure, has been treated with a disinfecting product containing active chlorine and coloring matter.

A further object of the invention is to provide a method of demonstrating the absence of the disinfectant after flushing of the liquid with water.

According to one aspect of the present invention, there is provided a method of producing a disinfecting or sterilizing liquid, cream, ointment, paste, or gel by dissolving in a solvent a stable ready-to-use dosage form containing coloring matter and an organic chloroxidizer acting as a chlorine donor releasing active chlorine; characterized in that the coloring compound in said coloring matter is in its maximal level of oxidation; and in that said disinfectant or sterilizing liquid, cream, ointment, paste, or gel has a pH of 4 - 8 and is present in a sufficient quantity to disinfect or sterilize the surface, vessel, or tissue, as required, while a desired colored appearance of the liquid, cream, ointment, paste, or gel is stably maintained over a period of time which exceeds the period of time that the disinfectant would be normally used for that purpose.

The present invention thus differs from the above-cited U.S. Patent No. 5,685,262 both in the pH range (4 - 8, i.e. from acidic to slightly alkaline), of the liquid obtained from the dosage form (a solid or semi-solid vs. a liquid), and in the use of an organic chloroxidizer vs. an inorganic hypochlorite solution.

According to another aspect of the invention, there is provided a method of indicating, by color, which surface or biological tissue, such as teats of a milking animal, or skin area before a medical procedure, has been treated with a disinfecting or sterilizing liquid, cream, ointment, paste, or gel, by applying to the surface or biological tissue a formulation produced by the above-described method.

According to yet a further aspect of the invention, there is provided a colored disinfectant liquid, cream, ointment, paste, or gel comprising a solid or semi-solid formulation dissolved in a solvent and containing an organic chloroxidizer acting as a chlorine donor releasing active chlorine, and coloring matter added thereto; characterized in that the coloring compound in said coloring matter is in its maximal level of oxidation; and in that said disinfectant or sterilizing liquid, cream, ointment, paste, or gel has a pH of 4 - 8 and is present in a sufficient quantity to disinfect or sterilize a surface, vessel, or tissue as required while the liquid, cream, ointment, paste, or gel stably maintains a desired colored appearance over a period of time which exceeds the period of time that the resultant product liquid would normally be used for that purpose.

In the preferred embodiments of the invention described below, the organic chloroxidizer is Troclosene (dichloro-s-triazinetrione) or its salts; and is present up to 5,000 ppm LAC (latent active chlorine). The coloring matter is in its maximal level of oxidation and is preferably selected from the group of ferric oxide saccharate, Prussian Blue and its derivatives, and titanium oxide; however, other suitable non-oxidisable compounds may be used.

Further features and advantages of the invention will be apparent from the description below.

The invention is herein described, by way of example, with reference to the following examples:

### BASE FORMULATION

The following examples were added to a solid or semi solid Troclosene (dichloro-s-triazinetrione) salt formulation, containing an organic acid and/or an alkaline salt for pH adjustment, which when dissolved in water or suitable aqueous solvent produced a disinfectant/sterilizing liquid with a concentration of between 150 and 3000 ppm L.A.C. (latent available chlorine) and a near neutral pH between 4 to 8.

### EXAMPLE 1

A brown colored liquid is obtained when ferric oxide saccharate is added to the base formulation. The colored liquid obtained when the resultant formulation is dissolved in water or suitable aqueous solvent, remains unchanged for a suitable extended period of time.

### EXAMPLE 2

A compound which may be varied in its hue between blue and green is obtained when chemicals which react stoichiometrically to form the characteristically insoluble Prussian Blue or a derivative thereof are admixed, dried and then added to the base formulation. The colored liquid obtained when the resultant formulation is dissolved in water or other suitable aqueous solvent remains unchanged and homogeneous for at least 24 hours.

### EXAMPLE 3

An opaque white smooth semi-solid preparation is obtained when titanium oxide is added to a formulation containing a Troclosene salt together with inert excipients.

In the foregoing examples, the Troclosene salt is present up to 5,000 ppm LAC, preferably in the range of 150 to 3,000 ppm L.A.C. Screening of the colored examples using this range of biocidal concentrations have not shown less biocidal activity than the base formulation of the same concentration without color.

Following are examples of many of the applications that liquids containing Troclosene monovalent salt, and prepared with coloring matter in accordance with the above-described method for examples 1 and 2, may be used:
- In dipping or spraying of the teats of mammals (such as cows, goats, buffalo, sheep, camels, Ilamas, yaks) prior to or after milking in the prevention of mastitis, wherein the color allows the operator to see which teats have been treated;
- In the cleaning of dairy apparatus, including formulations containing other ingredients such as, but not only, surfactants, wherein the color acts as a marker to show when the final rinse is satisfactorily completed;
- In the hygiene, disinfecting and sterilization of medical and allied apparatus, wherein a colored liquid is more desirable than a clear, colorless solution;
- In the cleaning of apparatus and surfaces used in the food and catering industry, including formulations containing other ingredients such as, but not only, surfactants, wherein the color acts as an indicating marker to highlight areas which are not in contact with the disinfecting liquid and also to show when the final rinse is satisfactorily completed;
- In the dipping of plant cuttings in agricultural and horticultural applications, wherein the color allows the operator to see which plants have been treated;
- The coloring of products containing organic chloroxidizing compounds, and in particular Troclosene monovalent salt, may also be used in swimming pools, ornamental pools, and similar applications; in the sanitation of water in vases or other containers in which cut flowers are placed in order to prolong their life; and in the coloring of the drinking water of fowls and mammals in order to show that the water was chlorinated.

Following are additional examples of applications containing Troclosene monovalent salt, with the coloring matter in accordance with the above-described methods for all the Examples 1, 2 and 3:
- In the antisepsis and cleaning of the skin in humans and animals prior to surgical or similar procedures, wherein the color acts as an indicator to delineate the areas which have been disinfected;
- In the antisepsis and cleaning of surfaces which are dark in color, and the white opaqueness of Example 3 enables the highlighting of areas which are not in contact with the disinfecting agent and also to show when the final rinse is satisfactorily completed.

The base formulation may be used in the form of:
- a soluble or soluble effervescent tablet;
- a measured quantity of soluble or soluble effervescent granules;
- a cream, ointment, paste, or gel;
- a liquid in a sealed bottle or sachet.

It will also be appreciated that the above-described method may also be used to indicate which surface or biological tissue, such as teats of a milking animal or skin area before a medical procedure, has been treated with the disinfectant liquid, cream, ointment, paste or gel; and also that the absence of the disinfectant after use may be indicated by flushing the surface or tissue with water until the absence of color is noted in the flushed water.

While the invention has been described with respect to several embodiments, it will be appreciated that these are set forth merely for purposes of example, and that many other variations, modifications and applications of the invention may be made.

## Claims

1. A method of producing a disinfectant or sterilizing liquid, cream, ointment, paste, or gel by dissolving in a solvent a stable ready-to-use solid or semi-solid dosage form containing coloring matter and an organic chloroxidizer acting as a chlorine donor releasing active chlorine; **characterized in that** said coloring matter is or includes a coloring compound selected from the group consisting of ferric oxide saccharate, Prussian Blue and titanium oxide; and **in that** said disinfectant or sterilizing liquid, cream, ointment, paste, or gel has a pH of 4 - 8 and is present in a sufficient quantity to disinfect or sterilize the surface, vessel, or tissue, as required, while a desired colored appearance of the liquid, cream, ointment, paste or gel is stably maintained over a period of time which exceeds the period of time that the disinfectant would be normally used for that purpose.

2. The method according to Claim 1, wherein the said solid or semi-solid dosage form is soluble in an aqueous solvent.

3. The method according to Claim 1, wherein said aqueous solvent is water.

4. The method according to Claim 1, wherein said chloroxidizer is Troclosen (dichloro-s-triazinetrione) or a salt thereof.

5. The method according to Claim 1, wherein said organic chloroxidizer is present up to 5,000 ppm LAC (latent active chlorine).

6. A method of indicating, by color, which surface or biological tissue, such as teats of a milking animal, or skin area before a medical procedure, has been treated with a disinfecting liquid, cream, ointment, paste, or gel, by applying to said surface or biological tissue a formulation produced by the method of claim 1.

7. A method of indicating the absence of the disinfectant after use according to the method of Claim 6, by flushing the surface or tissue with water until the absence of color is noted in the flush water.

8. A colored disinfectant liquid, cream, ointment, paste, or gel comprising a solid or semi-solid formulation dissolved in a solvent and containing an organic chloroxidizer acting as a chlorine donor releasing active chlorine, and coloring matter added thereto; **characterized in that** said coloring matter is or includes a coloring compound selected from the group consisting of ferric oxide saccharate, Prussian Blue and titanium oxide; and **in that** said disinfectant or sterilizing liquid, cream, ointment, paste, or gel has a pH of 4 - 8 and is present in a sufficient quantity to disinfect or sterilize a surface, vessel, or tissue as required, while the liquid, cream, ointment, paste or gel stably maintains a desired colored appearance over a period of time which exceeds the period of time that the resultant product liquid would normally be used for that purpose.

9. The colored disinfectant according to Claim 8, wherein said solid or semi-solid formulation is soluble in an aqueous solvent.

10. The colored disinfectant according to Claim 8, wherein said organic chloroxidizer is Troclosene (dichloro-s-triazinetrione).

11. The colored disinfectant according to Claim 8, wherein said organic chloroxidizer is present up to 5,000 ppm LAC (latent active chlorine).

12. The method of any one of claims 1 to 7 or the colored disinfectant of any one of claims 8 to 11, wherein said coloring matter is or includes a coloring compound selected from the group consisting of ferric oxide saccharate and Prussian Blue.

## Patentansprüche

1. Verfahren zur Herstellung einer Flüssigkeit, Creme, Salbe, Paste oder Gel zur Desinfizierung oder Sterilisierung durch Auflösen in einem Lösungsmittel einer stabilen, gebrauchsfertigen festen oder halbfesten Darreichungsform, enthaltend Färbemittel und ein organisches Chloroxidationsmittel, das als ein Chlorspender fungiert, der Aktivchlor abgibt; **dadurch gekennzeichnet, dass** das Färbemittel eine Färbeverbindung ist oder enthält, die ausgewählt ist aus der Gruppe bestehend aus Eisenoxidsaccharat, Preußischblau und Titanoxid; und dass die Flüssigkeit, Creme, Salbe, Paste oder das Gel zur Desinfizierung oder Sterilisierung einen pH-Wert von 4 - 8 aufweist und in einer ausreichenden Menge vorhanden ist, um die Oberfläche, den Behälter oder das Gewebe, je nach Bedarf, zu desinfizieren oder zu sterilisieren, während eine gewünschte farbliche Erscheinung der Flüssigkeit, Creme, Salbe, Paste oder des Gels über einen Zeitraum hinaus stabil gehalten wird, der den Zeitraum übersteigt, in dem das Desinfizierungsmittel normalerweise für diesen Zweck verwendet würde.

2. Verfahren nach Anspruch 1, wobei die feste oder halbfeste Darreichungsform in einem wässrigen Lösungsmittel löslich ist.

3. Verfahren nach Anspruch 1, wobei das wässrige Lösungsmittel Wasser ist.

4. Verfahren nach Anspruch 1, wobei das Chloroxidationsmittel Troclosen (Dichlor-s-triazintrion) oder ein Salz davon ist.

5. Verfahren nach Anspruch 1, wobei vom organischen Chloroxidationsmittel bis zu 5.000 ppm LAC (latentes Aktivchlor) vorhanden ist.

6. Verfahren des Anzeigens vor einem medizinischen Eingriff, nach Farbe, welche Oberfläche oder biologisches Gewebe, wie z.B. die Euter eines Melktiers, oder welcher Hautbereich mit einer Flüssigkeit, Creme, Salbe, Paste oder Gel zum Desinfizieren behandelt worden ist, durch Auftragen auf die Oberfläche oder auf das biologische Gewebe einer Formulierung, die nach dem Verfahren von Anspruch 1 hergestellt worden ist.

7. Verfahren des Anzeigens der Abwesenheit des Desinfektionsmittels nach der Verwendung gemäß dem Verfahren nach Anspruch 6, durch Spülen der Oberfläche oder des Gewebes mit Wasser, bis die Abwesenheit der Farbe im Spülwasser erkenntlich ist.

8. Gefärbte Flüssigkeit, Creme, Salbe, Paste oder Gel zum Desinfizieren, umfassend eine feste oder halbfeste Formulierung, die in einem Lösungsmittel aufgelöst ist und enthaltend ein organisches Chloroxidationsmittel, das als ein Chlorspender fungiert, der Aktivchlor abgibt, und dem ein Färbemittel hinzugefügt worden ist; **dadurch gekennzeichnet, dass** das Färbemittel eine Färbeverbindung ist oder enthält, die ausgewählt ist aus der Gruppe bestehend aus Eisenoxidsaccharat, Preußischblau und Titanoxid; und dass die Flüssigkeit, Creme, Salbe, Paste oder das Gel zur Desinfizierung oder Sterilisierung einen pH-Wert von 4 - 8 aufweist und in einer ausreichenden Menge vorhanden ist, um die Oberfläche, den Behälter oder das Gewebe, je nach Bedarf, zu desinfizieren oder zu sterilisieren, während eine gewünschte farbliche Erscheinung der Flüssigkeit, Creme, Salbe, Paste oder des Gels über einen Zeitraum hinaus stabil gehalten wird, der den Zeitraum übersteigt, in dem das Desinfizierungsmittel normalerweise für diesen Zweck verwendet würde.

9. Gefärbtes Desinfektionsmittel nach Anspruch 8, wobei die feste oder halbfeste Formulierung in einem wässrigen Lösungsmittel löslich ist.

10. Gefärbtes Desinfektionsmittel nach Anspruch 8, wobei das organische Chloroxidationsmittel Troclosen (Dichlor-s-triazintrion) ist.

11. Gefärbtes Desinfektionsmittel nach Anspruch 8, wobei vom organischen Chloroxidationsmittel bis zu 5.000 ppm LAC (latentes Aktivchlor) vorhanden ist.

12. Verfahren nach einem der Ansprüche 1 bis 7 oder gefärbtes Desinfektionsmittel nach einem der Ansprüche 8 bis 11, wobei das Färbemittel eine Färbeverbindung ist oder enthält, die ausgewählt ist aus der Gruppe bestehend aus Eisenoxidsaccharat und Preußischblau.

## Revendications

1. Procédé de production d'un liquide, d'une crème, d'un onguent, d'une pâte ou d'un gel désinfectant(e) ou stérilisant(e) par dissolution dans un solvant d'une formulation stable prête à l'emploi, solide ou semi-solide, renfermant une substance colorante et un chloroxydant organique agissant comme un donneur de chlore libérant du chlore actif ; **caractérisé en ce que** ladite substance colorante est ou comprend un composé colorant choisi dans le groupe constitué du saccharate d'oxyde ferrique, du Bleu de Prusse et de l'oxyde de titane ; et **en ce que** ledit (ladite) liquide, crème, onguent, pâte ou gel désinfectant(e) ou stérilisant(e) a un pH de 4 à 8 et est présent(e) en une quantité suffisante pour désinfecter ou stériliser la surface, le récipient ou le tissu, selon les besoins, tandis qu'un aspect coloré souhaité stable du liquide, de la crème, de l'onguent, de la pâte ou du gel est conservé pendant une période qui dépasse la période pendant laquelle le désinfectant est normalement utilisé à cette fin.

2. Procédé selon la revendication 1, dans lequel ladite formulation solide ou semi-solide est soluble dans un solvant aqueux.

3. Procédé selon la revendication 1, dans lequel ledit solvant aqueux est l'eau.

4. Procédé selon la revendication 1, dans lequel ledit chloroxydant est le Troclosene (dichloro-s-triazinetrione) ou un sel de celui-ci.

5. Procédé selon la revendication 1, dans lequel ledit chloroxydant organique est présent en une quantité maximale de 5 000 ppm de LAC (chlore actif latent).

6. Procédé servant à indiquer, à l'aide d'une couleur, quelle surface ou quel tissu biologique, tel que les trayons d'un animal de traite, ou la surface de la peau avant une procédure médicale, a été traité(e) par un liquide, une crème, un onguent, une pâte ou un gel désinfectant(e), en appliquant sur ladite surface ou ledit tissu biologique une formulation produite par le procédé selon la revendication 1.

7. Procédé servant à indiquer l'absence du désinfectant après utilisation selon le procédé de la revendication 6, en rinçant la surface ou le tissu à l'eau jusqu'à noter l'absence de couleur dans l'eau de rinçage.

8. Liquide, crème, onguent, pâte ou gel désinfectant(e) coloré(e) comprenant une formulation solide ou semi-solide dissoute dans un solvant et renfermant un chloroxydant organique agissant comme un donneur de chlore libérant du chlore actif, et une substance colorante y étant ajoutée ; **caractérisé en ce que** ladite substance colorante est ou comprend un composé colorant choisi dans le groupe constitué du saccharate d'oxyde ferrique, du Bleu de Prusse et de l'oxyde de titane ; et **en ce que** ledit (ladite) liquide, crème, onguent, pâte ou gel désinfectant(e) ou stérilisant(e) a un pH de 4 à 8 et est présent(e) en une quantité suffisante pour désinfecter ou stériliser une surface, un récipient ou un tissu, selon les besoins, tandis que le liquide, la crème, l'onguent, la pâte ou le gel conserve un aspect coloré souhaité stable pendant une période qui dépasse la période pendant laquelle le produit liquide résultant est normalement utilisé à cette fin.

9. Désinfectant coloré selon la revendication 8, dans lequel ladite formulation solide ou semi-solide est soluble dans un solvant aqueux.

10. Désinfectant coloré selon la revendication 8, dans lequel ledit chloroxydant organique est le Troclosene (dichloro-s-triazinetrione).

11. Désinfectant coloré selon la revendication 8, dans lequel ledit chloroxydant organique est présent en une quantité maximale de 5 000 ppm de LAC (chlore actif latent).

12. Procédé selon l'une quelconque des revendications 1 à 7 ou désinfectant coloré selon l'une quelconque des revendications 8 à 11, dans lequel ladite substance colorante est ou comprend un composé colorant choisi dans le groupe constitué du saccharate d'oxyde ferrique et du Bleu de Prusse.
